(19) Europäisches Patentamt
European Patent Office
Office européen des brevets

(11) Publication number: **0 161 425**
**A2**

(12) # EUROPEAN PATENT APPLICATION

(21) Application number: 85103318.3

(22) Date of filing: 21.03.85

(51) Int. Cl.⁴: **A 01 N 47/44**, A 01 N 43/40, C 11 D 3/48

(30) Priority: 23.03.84 US 592664

(43) Date of publication of application: 21.11.85 Bulletin 85/47

(84) Designated Contracting States: AT BE CH DE FR GB IT LI NL SE

(71) Applicant: STERLING DRUG INC., 90 Park Avenue, New York New York (US)

(72) Inventor: Gorman, William George, 25 Old Troy Road, East Greenbusch New York (US)
Inventor: Popp, Karl Frederick, 1775 Duck Pond Road, Schodack Landing New York (US)

(74) Representative: Baillie, Iain Cameron et al, c/o Ladas & Parry Isartorplatz 5, D-8000 München 2 (DE)

(54) **Antimicrobial surface degerming compositions.**

(57) Antimicrobial surface degerming compositions comprising an aryl, alkyl, or cycloalkyl bisbiguanide or a bis[4-(substitued-amino)-1-pyridinium]alkane, especially octenidine hydrochlorid, one or more quaternary ammonium phosphate ester surfactants and an aqueous vehicle and in a further aspect additionally comprising one or more of a polyethylene glycol ester surfactant and a quaternary nitrogen-containing cellulose ether, especially antimicrobial skin cleansing compositions.

EP 0 161 425 A2

## BACKGROUND OF THE INVENTION

The invention relates to antimicrobial surface dergerming compositions containing as the antimicrobial agent an aryl or alkyl bisbiguanide or a bis[4-(substituted-amino)-1-pyridinium]alkane and as the primary surfactant a quaternary ammonium phosphate ester, especially antimicrobial skin cleansing compositions, and method of use thereof.

U.S. Pat. 2,684,924 issued July 27, 1954 describes antimicrobial aryl bisbiguanides including chlorhexidine, which is also described by The Merck Index (Tenth Edition, 1983, monograph 2057) and of which a preferred salt form is the digluconate salt. U.S. Pat. 4,053,636 issued October 11, 1977 describes antimicrobial dichlorocyclopropylphenyl bisbiguanides and poloxalene-amine oxide surfactant compositions thereof. U.S. Pat. 3,468,898 issued September 23, 1969 describes antimicrobial alkyl bisbiguanides and states that they can be used "in aqueous detergent solutions".

Gundersen U.S. Pat. 4,022,834 describes antimicrobial cycloalkyl bisbiguanides.

U.S. Pat. 4,206,215 describes antimicrobial bis[4-(substituted-amino)-1-pyridinium]-alkanes and states that they "can be formulated with any compatible, pharmaceutically acceptable surfactant preferably a nonionic surfactant". Example 10 describes 1,10-bis[4-(octylamino)-1-pyridinium]decane dichloride, whose generic name is octenidine hydrochloride.

U.S. Pat. 4,209,449 describes quaternary ammonium phosphate ester surfactants and as examples of compositions thereof a tearless baby shampoo and a rinse conditioner. Certain of these surfactants are commercially available under the MONAQUAT tradename. The polyethylene glycol ester surfactants are a known class of surfactants of the nonionic type, specific examples of which are described by generic name (CTFA Adopted Name), trade name and structural formula by CTFA Cosmetic Ingredient Dictionary (Third Edition, 1982; published by The Cosmetic, Toiletry and Fragrance Association, Inc., 1110 Vermont Avenue, N.W., Washington, D.C. 20005) and are commercially available. U.S. Pat. 3,472,840 describes quaternary nitrogen-containing cellulose ethers, specific examples of which also are described by the above-cited CTFA Cosmetic Ingredient Dictionary and are commercially available. U.S. Pat. 4,420,484 describes antimicrobial skin cleansing compositions comprising an aryl or alkyl bisbiguanide and/or bis[4-(substituted-amino)-1-pyridinium]alkane antimicrobial agent and a polyethylene glycol ester surfactant - betaine and/or amine oxide surfactant combination. Compositions containing chlorhexidine gluconate and octenidine hydrochloride as the antimicrobial agent are specifically described.

The present invention deals with an antimicrobial surface degerming composition comprising

A) from about 0.01% to about 10% by weight of an antimicrobial agent selected from the group consisting of

a) a compound having the structural formula

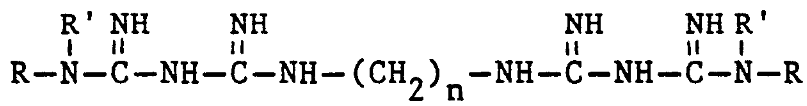

Formula I

wherein

R taken alone is phenyl substituted by methyl, methoxy, nitro or halo, p-(2,2-dichlorocyclopropyl)phenyl, alkyl having from 6 to 16 carbon atoms, cycloalkyl or polycyclic alkyl having 5 or more carbon atoms or lower-alkyl-cycloalkyl or cycloalkyl-lower-alkyl having from 1 to 4 carbon atoms in lower alkyl;

R' taken alone is hydrogen; or

R and R' taken together are 3-azabicyclo(3,2,2)nonyl; and

n is an integer from 3 to 9; or

a pharmaceutically acceptable acid addition salt thereof; and

b) a compound having the structural formula

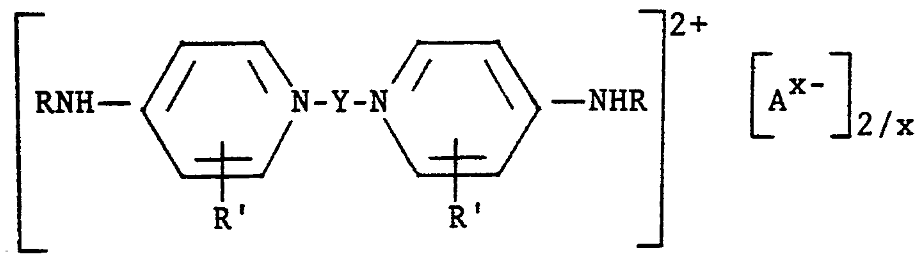

Formula II

wherein

Y is alkylene having from 4 to 18 carbon atoms and separating the two pyridinium groups by from 4 to 18 carbon atoms;

R is the same in both occurrences and is alkyl having from 6 to 18 carbon atoms, cycloalkyl having from 5 to 7 carbon atoms, benzyl or phenyl substituted by methylenedioxy or one or two substituents selected from the group consisting of halo, lower-alkyl, lower-alkoxy, nitro, cyano and trifluoromethyl;

R' is the same in both occurrences and is hydrogen or lower-alkyl;

$A^{x-}$ is a pharmaceutically acceptable anion; and

x is the valence of the anion;

B) from about 0.1% to about 50% by weight of one or more compounds having the structural formulas

a) 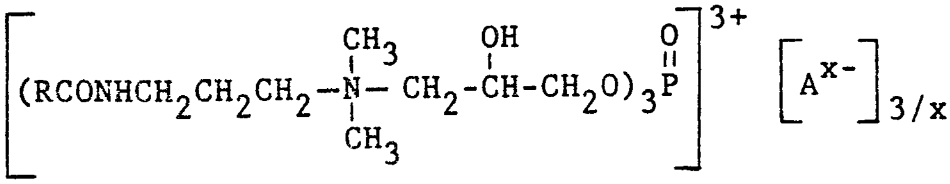

Formula III and

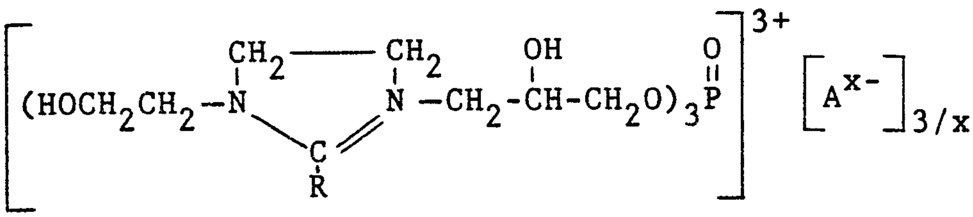

Formula IV

wherein

R is alkyl having from 5 to 17 carbon atoms;

$A^{x-}$ is a pharmaceutically acceptable anion; and

x is the valence of the anion; and

C) an aqueous vehicle.

If desired, the above-described composition additionally comprises in part B one or more compounds selected from the group consisting of

b) a compound having the structural formula

$RCOO-(CH_2CH_2O)_n-H$ Formula V,

$RCOO-(CH_2CH_2O)_n-COR$ Formula VI or

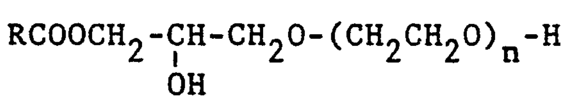 Formula VII

wherein

R is alkyl or alkenyl having from 8 to 20 carbon atoms or lanolin; and

n is an integer from about 8 to about 200; and

c) a compound having the structural formula

$$\left[R_{cell}(OR)_3\right] \qquad \text{Formula VIII}$$

wherein $R_{cell}$ is the residue of an anhydroglucose unit ($C_6H_{10}O_5$), x represents the degree of polymerization and is an integer from about 50 to about 20,000 and preferably from about 200 to about 5,000, the R's are the same or different and R has the structural formula

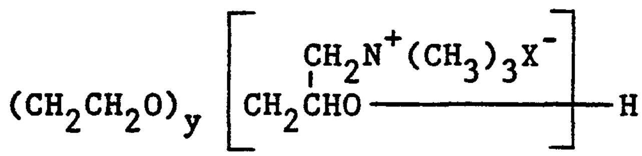

Formula IX

wherein y is an integer from 0 to 10, z is an integer from 0 to 3 and $X^-$ is a pharmaceutically acceptable anion.

One can degerm a living or nonliving surface by applying to the surface an antimicrobially effective amount of a composition according to either of the above-described composition aspects of the invention. The invention is especially useful in reducing bacterial counts on living mammal skin by applying to the skin an antimicrobially effective amount of the composition of the invention.

In Formula I halo is fluoro, chloro, bromo or iodo, preferably chloro. Alkyl is preferably hexyl, heptyl or 2-ethylhexyl. Cycloalkyl is preferably cyclohexyl. Lower-alkyl-cycloalkyl is preferably 4-methylcyclohexyl. Cycloalkyl-lower-alkyl is preferably cyclohexylmethyl. The preferred acid addition salts are the hydrochloride, acetate and gluconate salts. The preferred compound of Formula I is the digluconate salt of the compound wherein R is p-chlorophenyl and R' is hydrogen, whose generic name is chlorhexidine gluconate.

In Formula II Y can be branched or unbranched, preferably has from 8 to 12 carbon atoms, and is most preferably 1,10-decylene. When R is alkyl, it can also be branched or unbranched, preferably has from 7 to 9 carbon atoms, and is most preferably n-octyl. When R is cycloalkyl, it is preferably cyclohexyl. Halo is fluoro, chloro, bromo or iodo. When R is substituted phenyl, it is, for example, 3,4-methylenedioxyphenyl, p-chlorophenyl, p-tolyl, p-methoxyphenyl, p-nitrophenyl, m-cyanophenyl or m-(trifluoromethyl)phenyl. R' is preferably hydrogen. $A^{x-}$ is preferably chloride ion and x is thus preferably 1. In the preferred compound of Formula II Y is 1,10-decylene, R is n-octyl, R' is hydrogen, $A^{x-}$ is chloride ion, and x is 1. The generic name of this compound is octenidine hydrochloride.

In the compounds of Formulas III and IV R together with the carbon atom to which it is bonded is preferably the coconut acid radical (about 48% lauric, 18% myristic, 8% caprylic, 8% palmitic, 7% capric, 6% oleic, 3% linoleic and 2% stearic), $A^{x-}$ is chloride ion and x is therefore 1. The approved CTFA Adopted Name (not yet listed in the above-cited CTFA Cosmetic Ingredient Dictionary) of the compound of Formula III having these preferred R and $A^{x-}$ identities is Cocamidopropyl PG-Dimonium Chloride Phosphate. Of the compounds of Formula III and IV this is the preferred compound and is described as EXAMPLE #1 of above-cited U.S. Pat. 4,209,449. The compound of Formula lV wherein R together with the carbon atom to which it is bonded is the coconut acid radical is described in the same patent as EXAMPLE #9.

Representative compounds of Formulas V, VI and VII also have CTFA Adopted Names. PEG-150 Laurate is the compound of Formula V wherein R is undecyl and n has an average value of 150. PEG-150 Distearate is the compound of Formula VI wherein R is heptadecyl and n has an average value of 150. Of the compounds of Formulas V, VI and VII PEG-78 Glyceryl Cocoate of Formula VII wherein RCO is the coconut acid radical and n has an average value of 78 is the preferred compound.

The CTFA Adopted Name of the preferred compound of Formula VIII is Polyquaternium-10, particularly that which is sold under the trade name UCARE Polymer JR-30M.

The aqueous vehicle of the compositions of the invention is preferably water alone but can also include a pharmaceutically acceptable, water miscible organic diluent, for example, ethyl alcohol, isopropyl alcohol, propylene glycol or glycerin or a mixture thereof.

In addition to the above-described ingredients the compositions may also include pharmaceutical adjuncts, for example, humectants, emollients, lubricants, stabilizers, dyes, perfumes and preservatives. In spite of the presence of the antimicrobial agent a preservative may be necessary to prevent growth of microorganisms in the compositions. A pharmaceutically acceptable acid or base for pH adjustment and/or a pharmaceutically acceptable buffer for pH maintenance may also be added.

Living surfaces treated by the compositions of the invention include those of plants and animals, especially mammal skin, most especially human skin. Nonliving surfaces include hard and soft surfaces, for example, those of wood, metal, glass, ceramic, plastic, paper and cloth objects. The compositions are applied to the surfaces by conventional techniques including dipping, swabbing, spraying and scrubbing. The following examples illustrate the composition aspects of the invention.

## Example 1

| Ingredient | Percent by Weight |
|---|---|
| Octenidine Hydrochloride | 2.00 |
| Cocamidopropyl PG-Dimonium Chloride Phosphate | 5.00 |
| PEG-78 Glyceryl Cocoate | 10.0 |
| Perfume | 0.100 |
| Dye | 0.00500 |
| Sodium Hydroxide to make pH 7 | -- |
| Purified Water to make | 100.0 |

## Example 2

| Ingredient | Percent by Weight |
|---|---|
| Octenidine Hydrochloride | 2.00 |
| Cocamidopropyl PG-Dimonium Chloride Phosphate | 5.00 |
| Polyquaternium-10 (UCARE Polymer JR 30M) | 0.750 |
| Perfume | 0.100 |
| Dye | 0.00500 |
| Sodium Hydroxide to make pH 7 | -- |
| Purified Water to make | 100.0 |

## Example 3

| Ingredient | Percent by Weight |
|---|---|
| Octenidine Hydrochloride | 2.00 |
| Cocamidopropyl PG-Dimonium Chloride Phosphate | 6.0 |
| PEG-78 Glyceryl Cocoate | 11.0 |
| Sodium Phosphate | 0.276 |
| Disodium EDTA | 0.100 |
| Perfume | 0.100 |
| Dye | 0.00500 |
| Sodium Hydroxide to make pH 7.2 | -- |
| Purified Water to make | 100.0 |

## Example 4

| Ingredient | Percent by Weight |
|---|---|
| Octenidine Hydrochloride | 2.00 |
| Cocamidopropyl PG-Dimonium Chloride Phosphate | 6.0 |
| PEG-78 Glyceryl Cocoate | 11.0 |
| Isopropyl Alcohol | 5.00 |
| Sodium Phosphate | 0.276 |
| Disodium EDTA | 0.100 |
| Perfume | 0.100 |
| Dye | 0.00500 |
| Sodium Hydroxide to make pH 7.2 | -- |
| Purified Water to make | 100.0 |

Biological Properties of the Compositions

Excised Porcine Skin Disk Immersion Test

Sterile excised porcine skin disks 10 mm. in diameter were inoculated with a *Staphylococcus epidermidis* ATCC 17917 culture containing between $10^6$ and $10^7$ colony-forming units/ml., incubated in tryptose phosphate broth for 6 hr. at 37°C., separated from the broth, reincubated for 18-24 hr. at 30°C., washed twice with aqueous 0.0743 M potassium dihydrogen phosphate containing 0.1% of Triton X-100 brand of polyethylene glycol p-isooctylphenyl ether, and stored at -70°C. Each disk was then thawed, immersed for 3 min. in 2 ml. of one of the compositions of Examples 3 and 4 or in 25 mM aqueous potassium dihydrogen phosphate as a control solution, washed with 10 ml. of sterile distilled water for 5 sec., then sampled by swirling for 1 min. with 10 ml. of aqueous 0.025 M potassium dihydrogen phosphate containing 0.1% of Triton X-100 brand of polyethylene glycol p-isooctylphenyl ether, 0.2% lecithin, 1.55% Tween 80 brand of polysorbate 80, 1.0% Tamol-N Micro brand of condensed naphthalenesulfonic acid sodium salt anionic dispersant and 0.1% sodium thiosulfate.

To determine the number of surviving bacteria successive tenfold dilutions of 1.0 ml. aliquots of the sampling solution in aqueous 0.0035 M potassium dihydrogen phosphate containing 0.2% lecithin and 1.55% Tween 80 brand of polysorbate 80 were plated on soybean-casein

digest agar by the pour plate technique. The plates were incubated at 37°C. and counted. Mean $\log_{10}$ counts and reductions of counts from the control were determined and analyzed statistically. The following results, including standard errors, which were obtained using ten disks for each of the control solution and the tested examples, show that the compositions of Examples 3 and 4 caused significant mean $\log_{10}$ count reductions of bacteria on the disks.

| Example | Mean $\text{Log}_{10}$ Count of Bacteria Recovered/Disk | Mean $\text{Log}_{10}$ Count Reduction of Bacteria from Control/Disk |
|---|---|---|
| 3 | 4.77 ± 0.18 | 1.43 ± 0.18 |
| 4 | 4.58 ± 0.12 | 1.62 ± 0.12 |

Monkey Paw Scrub Test

The antimicrobial effect of the compositions of Examples 1-4 on the normal bacterial flora of the hands of anesthetized cynomolgous monkeys was measured. Each hand was tested separately and randomly and was first scrubbed for two minutes with Camay brand of bar soap in warm tap water, rinsed for one minute in running warm tap water, washed for two minutes with Camay brand of bar soap in warm tap water, rinsed for two minutes in running warm tap water, and dried with a sterile towel, then sampled for microflora before being scrubbed with the test composition.

Sampling was done by placing a sterile latex surgical glove over the hand, pouring into the glove 100 ml. of aqueous phosphate buffered (0.07 M, pH 7.8) sampling fluid containing Triton X-100 brand of polyethylene glycol p-isoctylphenyl ether (0.1%), massaging the hand in the sampling fluid for one minute, and diluting an aliquot (10 ml.) of the sampling fluid with an equal volume of aqueous phosphate buffered (pH 7.2) Tamol-N Micro brand of condensed naphthalenesulfonic acid sodium salt anionic dispersant (2%) neutralizer solution. The volume of the neutralized sample was thus 20 ml.

The hand was then washed for 45 seconds and scrubbed for 15 seconds with 5 ml. of the test composition, rinsed for 30 seconds under warm running tap water, washed for 90 seconds and scrubbed for 30 seconds with an additional 5 ml. of the test composition, rinsed again for 30 seconds under warm running tap water, dried with a sterile towel, and sampled again. This procedure was repeated three times for a total of four times during eight hours with a minimum of one hour between procedures (scrubs). No sample was taken after the third scrub.

Triplicate aliquots (1 ml. each) of each neutralized sample and each of three successive tenfold dilutions thereof were then plated by the pour-plate technique on soybean-casein digest agar. The plates were incubated aerobically at 37°C. for 48-72 hours, then counted using an automated colony counter (Model 880 Artek Systems Corp.)

interfaced with a computer (PDP 15), whereby the data were processed by program and are expressed below as mean $\log_{10}$ count reductions from after the bar soap scrub and before the first test composition scrub (Pre Scrub 1) to after the first (Post Scrub 1), second (Post Scrub 2) and fourth (Post Scrub 4) test composition scrubs and as overall mean $\log_{10}$ count reductions.

The following results including standard errors were obtained using 20 hands for each of Examples 1-2 and 24 hands for each of Examples 3-4 and show significantly and progessively greater reductions in microbial counts at each of the post scrub sampling times for each of the compositions.

| | Mean $\text{Log}_{10}$ Count Reduction from Pre Scrub 1 | | | |
|---|---|---|---|---|
| Example | Post Scrub 1 | Post Scrub 2 | Post Scrub 4 | Overall |
| 1 | 1.08 ± 0.10 | 1.45 ± 0.15 | 2.67 ± 0.26 | 1.73 |
| 2 | 1.11 ± 0.11 | 1.67 ± 0.18 | 2.82 ± 0.22 | 1.87 |
| 3 | 1.11 ± 0.11 | 1.70 ± 0.14 | 2.72 ± 0.24 | 1.84 |
| 4 | 0.86 ± 0.08 | 1.15 ± 0.15 | 2.24 ± 0.25 | 1.41 |

CLAIMS

1. An antimicrobial surface degerming composition comprising

A) from about 0.01% to about 10% by weight of an antimicrobial agent which is

a) a compound having the structural formula

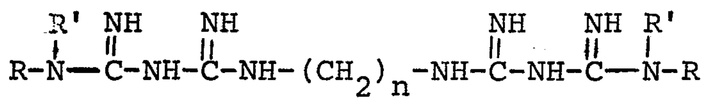

Formula I

wherein

R taken alone is phenyl substituted by methyl, methoxy, nitro or halo, p-(2,2-dichlorocyclopropyl)phenyl, alkyl having from 6 to 16 carbon atoms, cycloalkyl or polycyclic alkyl having 5 or more carbon atoms or lower-alkyl-cycloalkyl or cycloalkyl-lower-alkyl having from 1 to 4 carbon atoms in lower alkyl; and R' taken alone is hydrogen; or

R and R' taken together are 3-azabicyclo(3,2,2)nonyl; and

n is an integer from 3 to 9; or

a pharmaceutically acceptable acid addition salt thereof; or

b) a compound having the structural formula

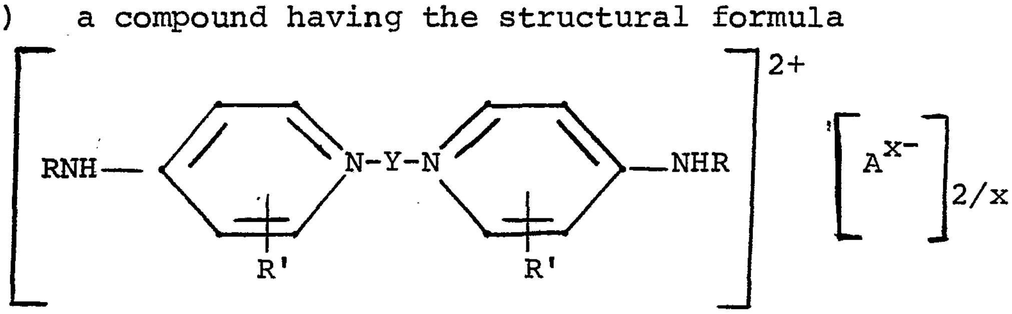

Formula II

wherein

Y is alkylene having from 4 to 18 carbon atoms and separating the two pyridinium groups by from 4 to 18 carbon atoms;

R is the same in both occurrences and is alkyl having from 6 to 18 carbon atoms, cycloalkyl having from 5 to

7 carbon atoms, benzyl or phenyl substituted by methylenedioxy or one or two substituents selected from the group consisting of halo, lower-alkyl, lower-alkoxy, nitro, cyano and trifluoromethyl;

R' is the same in both occurrences and is hydrogen or lower-alkyl;

$A^{x-}$ is a pharmaceutically acceptable anion; and

x is the valence of the anion;

B) from about 0.1% to about 50% by weight of one or more compounds having the structural formulas

a) 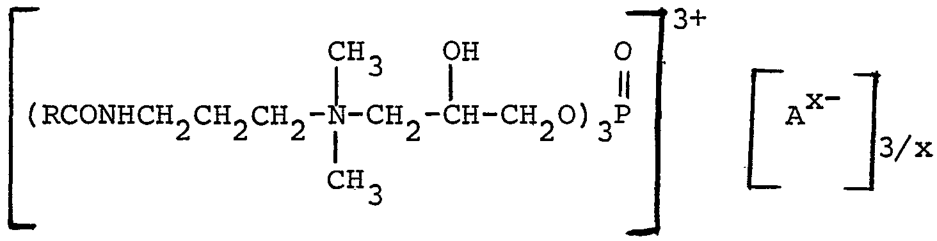

Formula III and

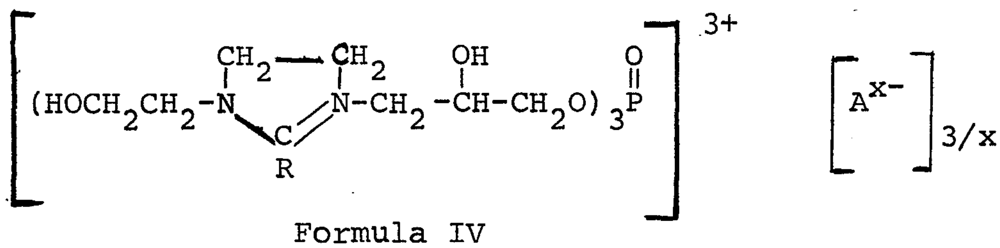

Formula IV

wherein

R is alkyl having from 5 to 17 carbon atoms;

$A^{x-}$ is a pharmaceutically acceptable anion; and

x is the valence of the anion; and

C) an aqueous vehicle.

2. A composition according to claim 1, wherein the compound of part A is a compound of Formula II.

3. A composition according to claim 2, wherein in the compound of Formula II Y is 1,10-decylene, R is n-octyl, R' is hydrogen, $A^{x-}$ is chloride ion, and x is 1, that is, wherein the compound of Formula II is octenidine hydrochloride.

4. A composition according to any one of the preceding claims, wherein the compound of part B is a compound of

Formula III.

5. A composition according to claim 4, wherein in the compound of Formula III RCO is the coconut acid radical, $A^{x-}$ is chloride ion, and x is 1, that is, wherein the compound of Formula III is Cocamidopropyl PG-Dimonium Chloride Phosphate.

6. A composition according to any one of the preceding claims, additionally comprising in part B one or more compounds selected from the group consisting of

b) a compound having the structural formula

$$RCOO-(CH_2CH_2O)_n-H \qquad \text{Formula V,}$$

$$RCOO-(CH_2CH_2O)_n-COR \qquad \text{Formula VI or}$$

$$RCOOCH_2-\underset{\displaystyle OH}{\underset{|}{CH}}-CH_2O-(CH_2CH_2O)_n-H \qquad \text{Formula VII}$$

wherein

R is alkyl or alkenyl having from 8 to 20 carbon atoms or lanolin; and

n is an integer from about 8 to about 200; and

c) a compound having the structural formula

$$\left[R_{cell}(OR)_3\right]_x \qquad \text{Formula VIII}$$

wherein $R_{cell}$ is the residue of an anhydroglucose unit $(C_6H_{10}O_5)$, x represents the degree of polymerization and is an integer from about 50 to about 20,000 and preferably from about 200 to about 5,000, the R's are the same or different and R has the structural formula

$$(CH_2CH_2O)_y\left[CH_2\overset{\displaystyle CH_2N^+(CH_3)_3X^-}{\overset{|}{C}}HO\right]_z H \qquad \text{Formula IX}$$

wherein y is an integer from 0 to 10, z is an integer from 0 to 3 and $X^-$ is a pharmaceutically acceptable anion.

7. A composition according to claim 6, wherein the additional compound is of Formula VII, particularly

wherein RCO is the coconut acid radical and n has an average value of 78, that is, wherein the compound of Formula VII is PEG-78 Glyceryl Cocoate.

8. A composition according to claim 6, wherein the additional compound is of Formula VIII, particularly Polyquaternium-10.

9. A composition according to any one of claims 6-8, comprising from about 1% to about 10% of the antimicrobial agent A), from about 5% to about 20% of the component B) and from about 5% to about 20% of the additional polyethylene glycol surfactant.

10. A method of degerming a living or nonliving surface which comprises applying to the surface an antimicrcbially effective amount of a composition according to any one of the preceding claims.

CLAIMS for Austria

1. An antimicrobial surface degerming composition not intended for curative purposes comprising

A) from about 0.01% to about 10% by weight of an antimicrobial agent which is

a) a compound having the structural formula

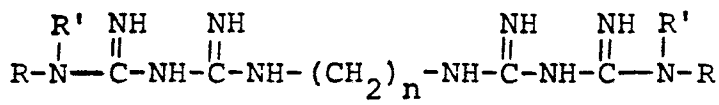

Formula I

wherein

R taken alone is phenyl substituted by methyl, methoxy, nitro or halo, p-(2,2-dichlorocyclopropyl)phenyl, alkyl having from 6 to 16 carbon atoms, cycloalkyl or polycyclic alkyl having 5 or more carbon atoms or lower-alkyl-cycloalkyl or cycloalkyl-lower-alkyl having from 1 to 4 carbon atoms in lower alkyl; and R' taken alone is hydrogen; or

R and R' taken together are 3-azabicyclo(3,2,2)nonyl; and

n is an integer from 3 to 9; or

a pharmaceutically acceptable acid addition salt thereof; or

b) a compound having the structural formula

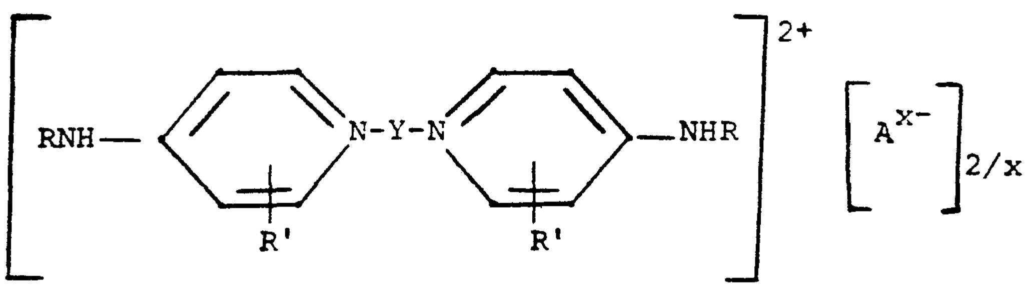

Formula II

wherein

Y is alkylene having from 4 to 18 carbon atoms and separating the two pyridinium groups by from 4 to 18 carbon atoms;

R is the same in both occurrences and is alkyl having from 6 to 18 carbon atoms, cycloalkyl having from 5 to

7 carbon atoms, benzyl or phenyl substituted by methylenedioxy or one or two substituents selected from the group consisting of halo, lower-alkyl, lower-alkoxy, nitro, cyano and trifluoromethyl;

R' is the same in both occurrences and is hydrogen or lower-alkyl;

$A^{x-}$ is a pharmaceutically acceptable anion; and

x is the valence of the anion;

B) from about 0.1% to about 50% by weight of one or more compounds having the structural formulas

a) 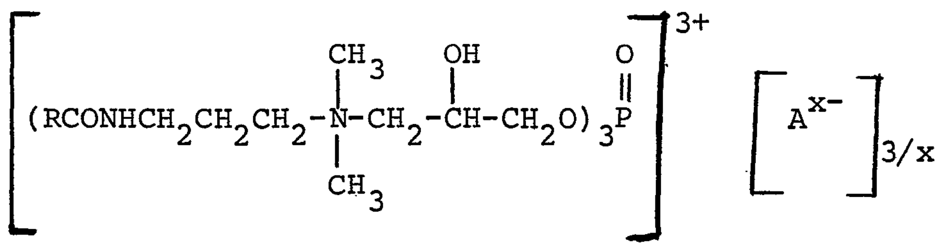

Formula III and

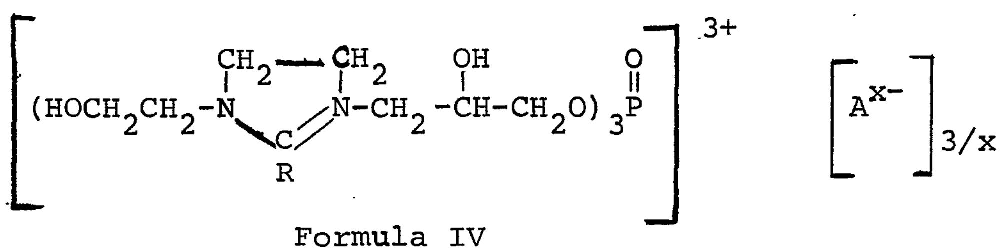

Formula IV

wherein

R is alkyl having from 5 to 17 carbon atoms;

$A^{x-}$ is a pharmaceutically acceptable anion; and

x is the valence of the anion; and

C) an aqueous vehicle.

2. A composition according to claim 1, wherein the compound of part A is a compound of Formula II.

3. A composition according to claim 2, wherein in the compound of Formula II Y is 1,10-decylene, R is n-octyl, R' is hydrogen, $A^{x-}$ is chloride ion, and x is 1, that is, wherein the compound of Formula II is octenidine hydrochloride.

4. A composition according to any one of the preceding claims, wherein the compound of part B is a compound of

Formula III.

5. A composition according to claim 4, wherein in the compound of Formula III RCO is the coconut acid radical, $A^{x-}$ is chloride ion, and x is 1, that is, wherein the compound of Formula III is Cocamidopropyl PG-Dimonium Chloride Phosphate.

6. A composition according to any one of the preceding claims, additionally comprising in part B one or more compounds selected from the group consisting of

b) a compound having the structural formula

$$RCOO-(CH_2CH_2O)_n-H \qquad \text{Formula V,}$$

$$RCOO-(CH_2CH_2O)_n-COR \qquad \text{Formula VI or}$$

$$RCOOCH_2-\underset{\displaystyle OH}{\underset{|}{CH}}-CH_2O-(CH_2CH_2O)_n-H \qquad \text{Formula VII}$$

wherein

R is alkyl or alkenyl having from 8 to 20 carbon atoms or lanolin; and

n is an integer from about 8 to about 200; and

c) a compound having the structural formula

$$\left[R_{cell}(OR)_3\right]_x \qquad \text{Formula VIII}$$

wherein $R_{cell}$ is the residue of an anhydroglucose unit $(C_6H_{10}O_5)$, x represents the degree of polymerization and is an integer from about 50 to about 20,000 and preferably from about 200 to about 5,000, the R's are the same or different and R has the structural formula

$$(CH_2CH_2O)_y\left[\begin{array}{l} \;\;\;\;\;\;CH_2N^+(CH_3)_3X^- \\ \;\;\;\;\;\;| \\ CH_2CHO \end{array}\right]_z H \qquad \text{Formula IX}$$

wherein y is an integer from 0 to 10, z is an integer from 0 to 3 and $X^-$ is a pharmaceutically acceptable anion.

7. A composition according to claim 6, wherein the additional compound is of Formula VII, particularly

wherein RCO is the coconut acid radical and n has an average value of 78, that is, wherein the compound of Formula VII is PEG-78 Glyceryl Cocoate.

8. A composition according to claim 6, wherein the additional compound is of Formula VIII, particularly Polyquaternium-10.

9. A composition according to any one of claims 6-8, comprising from about 1% to about 10% of the antimicrobial agent A), from about 5% to about 20% of the component B) and from about 5% to about 20% of the additional polyethylene glycol surfactant.

10. A method of degerming a living or nonliving surface which comprises applying to the surface an antimicrcbially effective amount of a composition according to any one of the preceding claims.